(19) Europäisches Patentamt
European Patent Office
Office européen des brevets

(11) **EP 4 686 466 A1**

(12) **EUROPEAN PATENT APPLICATION**

(43) Date of publication:
04.02.2026 Bulletin 2026/06

(21) Application number: 25190633.5

(22) Date of filing: 21.07.2025

(51) International Patent Classification (IPC):
*A61K 8/44* (2006.01)  *A61Q 19/08* (2006.01)
*A61K 31/198* (2006.01)  *A61P 17/00* (2006.01)
*A61P 29/00* (2006.01)

(52) Cooperative Patent Classification (CPC):
(C-Sets available)
**A61K 8/44; A61K 31/198; A61P 17/00;
A61P 29/00; A61Q 19/08;** A61K 2800/78;
A61K 2800/92                                    (Cont.)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: 30.07.2024  IT 202400017698

(71) Applicant: **Nutriresearch S.r.l.**
**20127 Milano (MI) (IT)**

(72) Inventor: **DIOGUARDI, Francesco Saverio**
**Milano (MI) (IT)**

(74) Representative: **Buzzi, Notaro & Antonielli d'Oulx
S.p.A.**
**Corso Vittorio Emanuele II, 6**
**10123 Torino (IT)**

(54) **A COMPOSITION CONTAINING AMINO ACIDS CAPABLE OF MODULATING THE IL-6 EXPRESSION**

(57)    A composition containing amino acids useful for modulating the IL-6 expression and counteracting epidermal aging and inflammatory processes.
Translation perfectly conforming to the original text.

EP 4 686 466 A1

(52) Cooperative Patent Classification (CPC): (Cont.)

C-Sets
**A61K 31/198, A61K 2300/00**

## Description

### Technical field

This invention refers to a composition containing amino acids capable of modulating the IL-6 expression and consequently counteracting the epidermal aging process.

### Technological background

Interleukin 6 (IL-6), also known as interferon β2, is a multifunctional cytokine with a key role in both the activation and deactivation of local and general inflammation. It regulates various physiological processes such as the synthesis of inflammatory acute-phase response proteins (serum amyloid a, c-reactive protein, proteases, prostaglandins, other cytokines, reactive oxygen species-ros), the immune response in its various mechanisms (it promotes the arrival of neutrophils in inflammatory sites, promotes the maturation of b lymphocytes and stimulates the production of immunoglobulins a, g and m, promotes the activation and differentiation of t lymphocytes), acts on hematopoiesis and cell growth by promoting the growth of blast cells by cooperating with IL-3.

It is expressed by different cells, T and B lymphocytes, monocytes, fibroblasts, keratinocytes, mesangial and endothelial cells and by cells of different tumors.

A large body of literature correlates aging and an increase in IL-6 [1], also induced by external factors such as UV [2]. Historically, however, some important data have questioned the uncritical univocity of this interpretation, because they describe the reduced capacity for immune response related to age due to excessive suppression of IL-6 under inflammatory stimulus [3], and the lack of influence of IL-6 on the production of keratinocyte growth factor (KGF) [4]. The data set therefore suggests that the IL-6 modulation, not the suppression of its production, is the best option to maintain an adequate capacity of epidermal cells to respond to environmental insults without suffering aggression and structural damage related to excessive inflammation, which among other things promotes excess cellular proliferation of keratinocytes and fibroblasts. This is a very modern concept, in fact only recently and under the pressure of studies on the reactivity of the immune system forced by the COVID19 pandemic, the inflammatory response has been included as an integral, indispensable and necessary part for an efficient immune response [5]. In this perspective, excessive suppression [1,2] as well as excessive expression and synthesis of IL-6 should be seen as not very advantageous extremes [3,4] even in specific cases such as the spread of the Herpes Simplex Virus in keratinocytes (HaCat) [3] as it is indispensable for the defensive immune response and the lesion healing. The possibility of modulating, or maintaining constant without making the inflammatory response to external agents excessive is therefore a fundamental objective to be achieved in the skin at any age, but particularly with advancing age.

Both the dermis and the epidermis show - in the case of aging - atrophy, reduction in thickness and ability to react to inflammatory stimuli of any kind; aging is particularly characterized by worsening of wound healing [3], a clinical condition proportional to the degree of inflammation and which is related to the reduction of the ability to generate an adequate number of fibroblasts [4].

Skin aging is, moreover, characterized in particular by the production of high levels of IL-6, as demonstrated by several models [6,7].

### Object and summary

An object of this invention is to provide a composition containing amino acids capable of reducing the inflammatory state at the epidermis level through modulation of the IL-6 expression so as to keep it close to physiological values.

An object of this invention is to provide a composition containing amino acids capable of balancing the keratinization process of the epidermis, by antagonizing any inflammatory states.

An object of this invention is to provide a composition containing amino acids capable of maintaining and/or increasing the collagen and elastin production in the epidermis.

An object of this invention is to provide a composition containing amino acids that can counteract the epidermal aging process, by nourishing it and preserving its proliferative capacity without compromising its immune response capacity.

According to one or more embodiments, these objects are achieved thanks to what is specifically referred to in the attached claims, which constitute an integral part of this disclosure.

In one embodiment, this invention relates to a composition containing amino acids comprising in a weight % to the total weight of the composition:

- branched amino acids about 37 ± 10%;

- basic amino acids about 31 ± 10%;
- amino acids with a polar chain about 11 ± 10%;
- neutral aromatic amino acids about 11 ± 10%;
- amino acids containing a sulfur atom about 10 ± 10%.

In one embodiment, this invention relates to a cosmetic use of the composition containing amino acids described herein to counteract the epidermal aging process.

### Detailed description of some exemplary embodiments

In the following description, numerous specific details are shown to provide a complete understanding of the embodiments. The embodiments may be practiced without one or more of the specific details, or with other methods, components, materials, etc. In other cases, well-known structures, materials, or operations are not shown or described in detail to avoid obscuring certain aspects of the embodiments.

Throughout this specification, reference to "an embodiment" or "one embodiment" means that a particular configuration, structure, or feature described in connection with the embodiment is included in at least one embodiment. Thus, the appearance of the phrases "in one embodiment" or "in a certain embodiment" at various places throughout this specification does not necessarily refer to the same embodiment. Furthermore, the particular configurations, structures, or features may be combined in any suitable way in one or more embodiments.

The headings and references used herein are merely for convenience and do not construe the scope or meaning of the embodiments.

The invention which is the subject of this application concerns a composition containing amino acids capable of modulating the inflammatory state at the epidermis level by acting on the IL-6 expression which is maintained close to physiological values (i.e. in a state of absence of inflammatory stimulus), consequently balancing the keratinization process without however increasing the duplication, and therefore the number of generations, of keratinocytes and fibroblasts. The composition containing amino acids according to this invention does not unnecessarily stimulate the immediate proliferation of keratinocytes and fibroblasts, which reduces the possibility - in the long term - of maintaining the number of cells intact; the present composition is therefore able to modulate the IL-6 expression at the fibroblast and keratinocyte level without using up the number of available generations of such cells (see US 5198465).

The presence of an age-related hyper-inflammatory state, or "inflamm-aging", characterized by high levels of IL-6 and therefore of TNF-alpha [1], has prompted the inventors to identify a composition containing amino acids capable of influencing in a limiting way the IL-6 expression under stimulation of TNF-alpha in keratinocytes. The reduction of IL-6 levels is in fact recognized as positive and has an anti-aging effect [2].

In one embodiment, this invention concerns a composition containing amino acids comprising, in a weight % to the total weight of the composition:

- branched amino acids about 37 ± 10%;
- basic amino acids about 31 ± 10%;
- amino acids with polar chain about 11 ± 10%;
- neutral aromatic amino acids about 11 ± 10%;
- amino acids containing a sulfur atom about 10 ± 10%.

In one embodiment, the branched amino acids leucine, isoleucine and valine are present in a ratio by weight leucine:isoleucine:valine, expressed as % to the total weight of the composition, of 18 ± 5% : 9 ± 5% : 10 ± 5%, preferably 18:9:10.

In one embodiment, the basic amino acids lysine, histidine and ornithine (as a salt with alpha-ketoglutarate) are present in a ratio by weight lysine:histidine:ornithine, expressed as % to the total weight of the composition, of 12 ± 5% : 12 ± 5% : 6.5 ± 5%, preferably 12:12:6.5.

In one embodiment, the amino acids with polar chain threonine and serine are present in a ratio by weight threonine:serine, expressed as % to the total weight of the composition, of 9 ± 5% : 2 ± 5%, preferably 9:2.

In one embodiment, the neutral aromatic amino acids tyrosine, phenylalanine and tryptophan are present in a ratio by weight tyrosine:phenylalanine:tryptophan, expressed as % to the total weight of the composition, of 4 ± 5% : 5 ± 5% : 2.5 ± 5%, preferably 4:5:2.5.

In one embodiment, the amino acids containing a sulfur atom methionine and cysteine (in the form of N-acetyl-cysteine) are present in a ratio by weight methionine:cysteine, expressed as % to the total weight of the composition, of 3 ± 5% : 7 ± 5%, preferably 3:7.

In one embodiment, the composition also comprises proline, preferably in a weight % to the total weight of the composition less than or equal to 2.5.

In one embodiment, the composition is free of arginine, glutamine and glutamic acid.

In one embodiment, the composition containing amino acids comprises leucine, isoleucine, valine, lysine, threonine, histidine, phenylalanine, tyrosine, methionine, cysteine (in the form of N-acetyl-cysteine), tryptophan, serine and ornithine (in the form of salt with alpha-keto (K)-glutarate). Testing of other mixtures characterized both by exclusion of individual amino acids and by inclusion of other amino acids did not yield the results unexpectedly observed for the composition of the present application.

In one embodiment, the composition comprises about 60% by weight of essential amino acids, where the essential amino acids are preferably represented by leucine, isoleucine, valine, lysine, histidine, threonine, phenylalanine, methionine and tryptophan.

In one embodiment, the composition comprises about 60% by weight of essential amino acids (leucine, isoleucine, valine, lysine, histidine, threonine, phenylalanine, methionine and tryptophan) and about 40% of non-essential amino acids (preferably represented by tyrosine, (N-acetyl-)cysteine, serine and ornithine, and possibly, but not necessarily, proline).

In one embodiment, the composition consists essentially of leucine, isoleucine, valine, lysine, histidine, threonine, phenylalanine, tyrosine, methionine, (N-acetyl)cysteine, tryptophan, serine and ornithine in the form of a salt with alpha-ketoglutarate.

In one embodiment, the branched amino acids are present in a weight % with respect to the total composition substantially equal to about 4 times the weight % of the amino acids with a polar chain.

In one embodiment, the branched amino acids are present in a weight % with respect to the total composition substantially equal to about 4 times the weight % of the aromatic amino acids.

In one embodiment, the amino acids with a polar chain are present in a weight % with respect to the total composition substantially equal to the weight % of the aromatic amino acids.

In one embodiment, the composition of this invention comprises in a weight % to the total weight of the composition:

- branched amino acids about 37;
- basic amino acids about 31;
- amino acids with a polar chain about 11;
- aromatic amino acids about 11;
- amino acids containing a sulfur atom about 10.

In one embodiment, the composition containing amino acids is suitable for oral or topical administration.

In one embodiment, the composition is in the form of a tablet, capsule, granules, cream, gel, and other formulations for topical use.

In one embodiment, the composition is suitable for oral administration in an amount between 1 and 24 g/day, depending on needs and body weight, preferably an amount between 1 and 15 g/day.

In one embodiment, the composition is suitable for topical administration at a concentration between 0.5% and 10%, preferably between 1% and 5%.

In one embodiment, this invention relates to a cosmetic use of a composition containing amino acids as described above to reduce the epidermal aging process through the IL-6 modulation.

In one embodiment, this invention relates to a cosmetic use of a composition containing amino acids as described above to stimulate collagen and/or elastin production in the dermis without this involving the stimulation of fibroblast duplication.

In one embodiment, this invention relates to a composition containing amino acids as described above for use in treating an inflammatory condition in a subject, preferably an inflammatory condition of the epidermis determined by external agents.

In one embodiment, the inflammatory condition is characterized by an overproduction of IL-6 in the epidermis under stimulation of external agents such as infrared rays, UVB rays.

The extracellular matrix (ECM) is made up of proteins capable of providing the necessary support to cells and tissues, which can be classified as structural or non-structural, depending on their function. Examples of structural proteins are collagen, elastin and fibronectin.

Collagen is the most abundant protein in the body and is found mainly in the ECM of connective tissues such as tendons and skin. It provides tensile strength but also plays a role in other cellular processes, such as adhesion and migration. Collagen is synthesized and secreted mainly by fibroblasts but also by endothelial cells and epithelial cells. There are at least 28 different types. In the ECM, collagen is arranged in fibrils to provide the structural integrity required for tissues. In particular, the fibrils characterize only collagen types I, II, III, V and XI, which in turn are tightly regulated by other ECM molecules, such as decorin and fibromodulin. In terms of distribution, type I collagen is the predominant form and widely present in almost all tissues. The presence of other types of collagen is more limited: type II collagen is distributed in cartilage and the cornea, type III collagen is the main form within the walls of blood vessels.

Elastin is the second most secreted structural protein, providing elasticity to tissues such as the dermis of the skin, with the ability to recover from continuous stretching. It is composed of individual tropoelastin subunits directly secreted into the extracellular space and then assembled into elastic fibers. The latter are also formed by an external layer of fibrillin microfibrils, with an essential role in membrane integrity.

The evaluation of the effects on the dermis underlying the epidermis, therefore on the fibroblasts, of the composition containing amino acids, subject of this disclosure, originates from the fact that skin aging depends on both components, keratinocytes and fibroblasts. That the number of fibroblasts is a sign of aging, and therefore depends on the limited number of generations available to fibroblasts, and that inflammation also causes aging due to the exhaustion of the capacity to maintain the number of fibroblasts intact is a well-known fact [Zorina, A.; Zorin, V.; Kudlay, D.; Kopnin, P. "Age-Related Changes in the Fibroblastic Differon of the Dermis: Role in Skin Aging" Int. J. Mol. Sci. 2022, 23, 6135 - https://doi.org/10.3390/ijms23116135].

The demonstration that the composition, subject of this disclosure, is able to facilitate the synthesis of collagen in a balanced way with elastin, therefore to increase the efficiency and the restoration of structural functions also by dermal fibroblasts [1], constitutes proof of the effectiveness of the composition in counteracting the aging process of the whole epidermis, dermis plus epidermis [2,3].

## Materials and Methods

Table 1 indicates the quantities expressed as percentages by weight of a preferred embodiment of the composition subject of this disclosure.

**Table 1**

| Amino acid | Comp. Weight % |
|---|---|
| Leucine | 18 |
| Isoleucine | 9 |
| Valine | 10 |
| Lysine | 12 |
| Threonine | 9 |
| Histidine | 12 |
| Phenylalanine | 5 |
| Tyrosine | 4 |
| Methionine | 3 |
| Acetyl-Cysteine | 7 |
| Tryptophan | 2.5 |
| Serine | 2 |
| Ornithine-$\alpha$KetoGlutarate | 6.5 |
| Proline | - |

The evaluation tests were performed in a blinded manner at UB-CARE S.r.l. (https://www.ub-careitaly.it/), an academic Spin-off of the University of Pavia, laboratories of the Immunology and General Pathology Unit, Department of Molecular Medicine, Via Ferrata 9, 27100-Pavia and in the laboratories, located in Via della Scienza, 12/14 Prado (PV) 27010 Pavia.

The MTT test is a colorimetric cytotoxicity assay that allows testing cell proliferation and viability based on the efficiency of mitochondrial respiration. MTT (3-(4,5-dimethylthiazol-2-yl)-2,5-diphenyl tetrazolium bromide) is a tetrazolium salt that is reduced in viable cells by the action of mitochondrial dehydrogenase. The reduction of MTT determines the formation of formazan crystals and the subsequent passage in isopropanol, attributing the characteristic purple coloration to the mitochondria of vital cells. On the contrary, in suffering or dead cells, therefore having inactive mitochondria, MTT will not be reduced with a consequent less intense or absent purple coloration (Mosman, 1983 [1]). Although it is therefore an assay that evaluates cellular respiration, the MTT test is also considered an excellent method for determining cell viability.

To set up the assay, the cells, a human keratinocyte line (HaCaT, code BS CL 168) provided by I.Z.L.E.R. (Istituto Zooprofilattico della Lombardia e Emilia-Romagna), were seeded homogeneously in a 96-well plate, and incubated at 37°C, with 5% $CO_2$. 24 h after seeding, cells were treated with the composition of this invention, starting from a concentration of 10 mg/ml and following scalar dilutions of 1:2 (preliminary tested range 0.078 - 10 mg/ml).

Cells treated with Sodium Lauryl Sulphate (SLS) were used as a positive control (tested range 0.039 - 5 mg/ml). Untreated cells were used as a negative control. The experiment was conducted in triplicate for each condition tested.

At the end of the treatment (24 h), cells were examined under an optical microscope, Leica Microsystem DM1000 Cytology, to record any morphological alterations due to a cytotoxic activity of the product under examination.

Subsequently, a solution of MTT was added to each well for 2 h at 37°C. The medium was then removed and the formazan crystals were solubilized with 100 μl of isopropanol. Subsequently, spectrophotometric reading was performed with a microplate reader at a wavelength of 570 nm (Agilent BioTek 800TS, filter 570nM, Part Number:7334570). Cell viability was calculated by measuring the difference in optical density between each of the tested concentrations compared to the negative control (untreated cells) [1].

The results are expressed in terms of cell viability:

Cell viability (%) = [OD(570 nm) test product/OD(570 nm) control] x 100

A reduction in cell viability greater than 30% is considered a cytotoxic effect.

The IL-6 expression levels after treatment with the composition containing amino acids described herein was evaluated on dermal keratinocytes using an ELISA kit (Diaclone, Human IL-6 ELISA Kit, # 950.030.096 1 x 96 - precoated).

For the test, the composition containing amino acids, evaluated in a blinded manner against other compositions differing in amino acid ratios, was dissolved in complete medium at 0.5% FBS, at a concentration of 5 mg/ml.

To facilitate its solubilization, the composition containing amino acids was sonicated for 30 min.

At the end of the treatment, the supernatants were collected and used for coating on a previously treated ELISA plate, provided by the kit.

The standards were reconstituted with distilled water and used to construct the standard curve.

The samples were added to each well in duplicate and the assay was performed according to the instructions provided by the kit.

The absorbance was then read at 450 nm.

Normal human embryonic fibroblasts (NHF, BS code CL 97) were cultured under completely sterile conditions and maintained in an incubator at 37°C with a 5% carbon dioxide ($CO_2$) atmosphere.

The list of culture media and reagents used is reported in Table 2.

**Table 2**

| Reagents/culture media | Supplier |
|---|---|
| Minimum Essential Medium (MEM) | Biowest |
| Penicillin-Streptomycin | Capricorn Scientific |
| Fetal Bovine Serum (FBS) | Gibco |
| Sodium Pyruvate | Sigma Aldrich |

For the preparation of the collagen assay, cells were uniformly seeded in 24-well plates (Thermo Fisher Scientific) at a density of 6 x $10^4$ cells per well and incubated at 37°C, with a humidified atmosphere of 5% $CO_2$. After 48 h, the two concentrations of the composition containing amino acids shown to be non-cytotoxic and with the best solubility were chosen to be tested in this assay.

Cells were homogeneously seeded in a 6-well plate and incubated at 37 °C, with a humidified atmosphere of 5% $CO_2$. The two highest concentrations shown to be non-cytotoxic and with the best solubility after the MTT assay were chosen for the 24 h treatment.

At the end of treatment, the supernatant of each sample was collected in sterile tubes and incubated overnight with Collagen Isolation & Concentration reagent (provided with the SircolTM, Soluble Collagen Assay S-1000 kit, Biocolor Life Science Assays). The following day, soluble collagen synthesis was measured several times using the commercial kit according to the manufacturer's instructions (Sircol™, Soluble Collagen Assay S-1000 and rechecked with Sircol™ 2.0, data reported, Biocolor Life Science Assay, https://www.biocolor.co.uk/).

For the preparation of the elastin assay, cells were homogeneously seeded in 25 $cm^2$ flasks at a density of 7 x $10^5$ cells/flask and incubated at 37°C, with a humidified atmosphere of 5% $CO_2$. After 48 h, the two concentrations of the composition containing amino acids under examination that were found to be non-cytotoxic and with the best solubility were chosen to be tested in this assay.

At the end of the incubation (24 h incubation), the cells were detached with a cell dissociation solution and diluted 3:1 with a 1 M oxalic acid solution (final concentration 0.25 M). Subsequently, the samples were heated at 95-100°C for 1 h, shaking them at regular intervals, and then the measurement of the elastin produced by each sample was performed following the manufacturer's instructions (Fastin™ Fastenˢ, Elastin Assay, Biocolor Life Science Assay, https://www.biocolor.co.uk/).

### Results

After exposure to the composition described herein, the respective cell viability at 24 h was evaluated, and the levels of IL-6 were tested with ELISA method on human keratinocytes (HaCaT) treated with TNF$\alpha$ in vitro, and the mixture of amino acids in Table 1 was tested at concentrations of 1.25 and 2.5 mg/mL. Higher concentrations (5 and 10 mg/mL) were found to be excessively osmolar and slightly cytotoxic, and also compromised > 30% cell viability (MTT), and this result is already unexpected and innovative, and has important implications regarding the use of higher concentrations of each substance capable of osmolar activity because they are soluble, an evaluation and data too often ignored.

Since a part of the amino acid mixtures, even if small, as reported in the literature, passes into the dermis, we wanted to verify the effects of the composition of table 1 also on immortalized human dermal fibroblasts, measuring both the production of IL-6 under LPS stimulation, but also the respective influence on collagen production, comparing the production of these molecules to untreated controls and to controls treated with ascorbic acid at a concentration of 12.5 $\mu$g/mL. For simplicity, the data will be reported to the mean datum of the result of the controls (untreated = CTRL) made 100.

The results are presented for HaCaT and Fibroblasts in separate paragraphs, even if their interpretation is obviously connected.

The data reported in table 3 correspond to the HaCaT cell viability assessed with MTT after exposure to TNF$\alpha$, control treated with TNF$\alpha$ (CTRL = 100$\pm$1). The tested concentrations of the composition containing amino acids in table 1 were 1.25 and 2.5 mg/mL. For simplicity, the values were rounded to the first decimal place.

**Table 3**

| Concentration | Cell viability |
|---|---|
| 1.25 mg/mL | 90.515 |
| 2.5 mg/mL | 82$\pm$4 |

Table 4 shows the quantities (pg/ml) of interleukin 6 (IL-6) measured by ELISA method after exposure to TNF$\alpha$, on 5 samples. Mean $\pm$ SD. Control treated with TNF$\alpha$ (CTRL= 100$\pm$9). The asterisk * identifies a significance < or << at p<0.01 evaluated with Student's T test against the control.

**Table 4**

| Concentration | IL-6 |
|---|---|
| 1.25 mg/mL | *82$\pm$11 |
| 2.5 mg/mL | *61$\pm$13 |

Table 5 shows the values of cell viability at 24 hours, measured by MTT, on immortalized human dermal fibroblasts. Control (CTRL) = 100. N tests = 5. The data reported for the composition containing amino acids at concentrations of 1.25 and 2.5 mg/mL were repeated, and those reported are simultaneous and carried out under the same conditions in which the studies on IL-6 and other collagen synthesis parameters were performed. * identifies statistical difference evaluated with T test compared to control or A with a value of P <0.001.

**Table 5**

| | Cell viability |
|---|---|
| CTRL = 100% | |
| 1.25mg/mL | 93$\pm$3 |

(continued)

|  | Cell viability |
| --- | --- |
| CTRL = 100% |  |
| 2.5mg/mL | *84±1 |

Table 6 shows the IL-6 values (pg/ml) after treatment with LPS added in the last 4 h of incubation in the controls and after treatment with the composition containing amino acids. The IL-6 value of the untreated control (CTRL) compared to the LPS-treated one is less than 1%. Vitamin C 50 $\mu$g/mL. N test =3. The standard deviation = 0 indicates values <0.5.

**Table 6**

|  | Vit. C | Composition |
| --- | --- | --- |
| CTRL = -99.2% |  |  |
| CTRL+LPS = 100 |  |  |
| 0.625 mg/mL | -7±0 | *-20±4 |

Table 7 reports collagen 1-A1 values as a % change from the LPS-treated control. The standard deviation has been omitted for convenience (=0) when < 0.5. The addition of LPS suppresses collagen type 1-A1 synthesis significantly (* = P<0.05 or less). The responses under Vitamin C administration are unchanged from the control. The composition, subject of this application, protected and maintained the production capacity of fibroblasts stimulated with LPS significantly at a concentration of 0.625 mg/mL. Of note, there is no correlation (r=0.33) between IL-6 values and collagen type 1-A1 production, and this is another unexpected and innovative result.

**Table 7**

|  | Vit. C | Composition |
| --- | --- | --- |
| CTRL + LPS = 100 |  |  |
| 0.625 mg/mL | -2.5±1 | *+33.5±3 |

Table 8 shows the variations in elastin compared to the control treated with LPS (0.94 ± 0.07 $\mu$g/mL) set at 100. The untreated control (CTRL): 0.78 ± 0.04 $\mu$g/mL, - 17% compared to the CTRL treated with LPS. The asterisk * indicates significance P < 0.05 compared to the CTRL + LPS. N = 3. Vitamin C is irrelevant, even if it shows a tendential but not significant decrease in elastin production under LPS stimulation. The composition described here, however, shows a significant increase in elastin production (P < 0.004).

**Table 8**

|  | Vit. C | Composition |
| --- | --- | --- |
| CTRL +LPS = 0.94±0.07 |  |  |
| 6.25 $\mu$g/mL | 0.88±0,04 |  |
| 0.625 mg/mL |  | *1.18±0.04 |

The mathematical relationship between the average values of collagen 1-A1 (COL1-A1) and elastin, multiplied by 100 and used as absolute numbers, assumes a value of 1.06±0.01 for the composition containing vitamin C and 0.97 ±0.04 for the composition containing amino acids described here. From these values, it can be deduced that the composition described here maintains a correct ratio between collagen and elastin even under inflammatory stimulus, preventing an imbalance that would make the skin inelastic and fibrotic. To our knowledge, a similar evaluation has never been made before this observation for any other product that can be used to protect the dermis from this and other inflammatory stimuli.

## REFERENCES

1. Kovacs EJ. Aging, traumatic injury, and estrogen treatment. Exp Gerontol. 2005 Jul;40(7):549-55. doi: 10.1016/j.exger.2005.04.009. PMID: 15975753.

2. Park CH, Lee MJ, Ahn J, Kim S, Kim HH, Kim KH, Eun HC, Chung JH. Heat shock-induced matrix metalloproteinase (MMP)-1 and MMP-3 are mediated through ERK and JNK activation and via an autocrine interleukin-6 loop. J Invest Dermatol. 2004 Dec;123(6):1012-9. doi: 10.1111/j.0022-202X.2004.23487.x. PMID: 15610507.

3. Aggarwal BB, Totpal K, LaPushin R, Chaturvedi MM, Pereira-Smith OM, Smith JR. Diminished responsiveness of senescent normal human fibroblasts to TNF-dependent proliferation and interleukin production is not due to its effect on the receptors or on the activation of a nuclear factor NF-kappa B. Exp Cell Res. 1995 May;218(1):381-8. doi: 10.1006/excr.1995.1169. PMID: 7737374.

4. Tang A, Gilchrest BA. Regulation of keratinocyte growth factor gene expression in human skin fibroblasts. J Dermatol Sci. 1996 Jan;11(1):41-50. doi: 10.1016/0923-1811(95)00418-1. PMID: 8867766.

5. Dobson GP, Biros E, Letson HL, Morris JL. Living in a Hostile World: Inflammation, New Drug Development, and Coronavirus. Front Immunol. 2021 Jan 22;11:610131. doi: 10.3389/fimmu.2020.610131. PMID: 33552070; PMCID: PMC7862725.

6. Il'yasova D, Colbert LH, Harris TB, Newman AB, Bauer DC, Satterfield S, Kritchevsky SB. Circulating levels of inflammatory markers and cancer risk in the health aging and body composition cohort. Cancer Epidemiol Biomarkers Prev. 2005 Oct;14(10):2413-8. doi: 10.1158/1055-9965.EPI-05-0316. PMID: 16214925.

7. Su H, Lei C-T and Zhang C (2017) Interleukin-6 Signaling Pathway and Its Role in Kidney Disease: An Update. Front. Immunol. 8:405. doi: 10.3389/fimmu.2017.00405

8. Ataie-Kachoie P, Pourgholami MH, Richardson DR, Morris DL. Gene of the month: Interleukin 6 (IL-6). J Clin Pathol. 2014 Nov;67(11):932-7. doi: 10.1136/jclinpath-2014-202493. Epub 2014 Jul 16. PMID: 25031389.

9. Kishimoto T. Interleukin-6: discovery of a pleiotropic cytokine. Arthritis Research & Therapy 2006, 8(Suppl 2):S2(doi:10.1186/ar1916)

10. Rana H, Truong NR, Johnson B, Baharlou H, Herbert JJ, Kandasamy S, et al. (2024) Herpes simplex virus spreads rapidly in human foreskin, partly driven by chemokine-induced redistribution of Nectin-1 on keratinocytes. PLoS Pathog 20(6): e1012267. https://doi.org/10.1371/journal. ppat.1012267

11. Wiley, C.D., Velarde, M.C., Lecot, P., Liu, S., Sarnoski, E.A., Freund, A., Shirakawa, K.,Lim, H.W., Davis, S.S., Ramanathan, A., Gerencser, A.A., Verdin, E., Campisi, J. Cell Metabolism 23, 303-314 February 9, 2016 http://dx.doi. org/10.1016/j.cmet.2015.11.011

12. R.M. Salama, M.A. Omar. Anti-aging effect of nifuroxazide on skin changes of aged male rat models via modulating immunoreactivity of IL-6/NF-κB/Caspase-3.Morphologie,107,359,2023.100605,ISSN 1286-0115, https://doi.org/10.1016/j.morpho.2023.06.001.

13. Gosain, A., DiPietro, L.A. Aging and wound healing. World J. Surg. 2004.28, 321-326.

14. Franceschi, C., Bonafe, M., Valensin, S., Olivieri, F., De Luca, M., Ottaviani, E., De Benedictis, G., 2000. Inflamm-aging. An evolutionary perspective on immunosenescence. Ann. NY Acad. Sci. 908, 244-254.

15. Kovacs JE. Aging, traumatic injury, and estrogen treatment. Experimental Gerontology 40 (2005) 549-555

16. Gendrisch F, Esser PR, Schempp CM, Wölfle U. Luteolin as a modulator of skin aging and inflammation. Biofactors. 2021 Mar;47(2):170-180. doi: 10.1002/biof.1699. Epub 2020 Dec 25. PMID: 33368702.

17. Boismal F, Serror K, Dobos G, Zuelgaray E, Bensussan A, Michel L. Vieillissement cutané - Physiopathologie et therapies innovantes [Skin aging: Pathophysiology and innovative therapies]. Med Sci (Paris). 2020 Dec;36(12):1163-1172. French. doi: 10.1051/medsci/2020232. Epub 2020 Dec 9. PMID: 33296633.

18. Jin T, Li L, Siow RC, et al. Collagen matrix stiffness influences fibroblast contraction force. Biomed Phys Eng Express 2016 ; 2 : 047002.

19. Sriram R, Gopal V. Aging Skin and Natural Bioactives that Impede Cutaneous Aging: A Narrative Review. Indian J Dermatol. 2023 Jul-Aug; 68(4): 414-424. Doi: 10.4103/ijd.ijd_932_22: 10.4103/ijd.ijd_932_22

20. Mosmann, T. (1983). Rapid colourimetric assay for cellular growth and survival: Application to proliferation and cytotoxicity assays. Journal of Immunological Methods, 65(1-2), 55-63.

21. van Meerloo J, Kaspers GJ, Cloos J. Cell sensitivity assays: the MTT assay. Methods Mol Biol. 2011;731:237-45. doi: 10.1007/978-1-61779-080-5_20.PMID: 21516412.

**Claims**

1. A composition containing amino acids comprising in a weight % to the total weight of the composition:

   - branched amino acids about 37 $\pm$ 10%;
   - basic amino acids about 31 $\pm$ 10%;
   - amino acids with polar chain about 11 $\pm$ 10%;
   - neutral aromatic amino acids about 11 $\pm$ 10%;
   - amino acids containing a sulfur atom about 10 $\pm$ 10%.

2. The composition according to claim 1, wherein the branched amino acids leucine, isoleucine and valine are present in a ratio by weight leucine:isoleucine:valine, expressed as % to the total weight of the composition, of 18 $\pm$ 5% : 9 $\pm$ 5% : 10 $\pm$ 5%.

3. The composition according to claim 1 or claim 2, wherein the basic amino acids lysine, histidine and ornithine are present in a ratio by weight lysine:histidine:ornithine, expressed as % to the total weight of the composition, of 12 $\pm$ 5% : 12 $\pm$ 5% : 6.5 $\pm$ 5%.

4. The composition according to any one of the preceding claims, wherein the amino acids with polar chain threonine and serine are present in a ratio by weight threonine:serine, expressed as % to the total weight of the composition, of 9 $\pm$ 5% : 2 $\pm$ 5%.

5. The composition according to any one of the preceding claims, wherein the neutral aromatic amino acids tyrosine, phenylalanine and tryptophan are present in a ratio by weight tyrosine:phenylalanine:tryptophan, expressed as % to the total weight of the composition, of 4 $\pm$ 5% : 5 $\pm$ 5% : 2.5 $\pm$ 5%.

6. The composition according to any one of the preceding claims, wherein the amino acids containing a sulfur atom methionine and cysteine are present in a ratio by weight methionine:cysteine, expressed as % to the total weight of the composition, of 7 $\pm$ 5% : 3 $\pm$ 5%.

7. The composition according to any one of the preceding claims, wherein the composition is free of arginine, glutamine and glutamic acid.

8. The composition according to any one of the preceding claims, wherein the branched amino acids are present in a weight % with respect to the total composition substantially equal to about 2 times the weight % of the amino acids with a polar chain.

9. The composition according to any one of the preceding claims, wherein the branched amino acids are present in a weight % with respect to the total composition substantially equal to about 4 times the weight % of the aromatic amino acids.

10. The composition according to any one of the preceding claims, wherein the amino acids with a polar chain are present in a weight % with respect to the total composition substantially equal to the weight % of the aromatic amino acids.

11. The composition according to any one of the preceding claims, comprising in a weight % to the total weight of the composition:

- branched amino acids about 37;
- basic amino acids about 31;
- amino acids with a polar chain about 11;
- aromatic amino acids about 11;
- amino acids containing a sulfur atom about 10.

12. The composition according to any one of the preceding claims, wherein the composition is suitable for oral administration in an amount between 1 and 24 g/day.

13. The composition according to any one of the preceding claims, wherein the composition is suitable for topical administration at a concentration between 0.5% and 10%.

14. A cosmetic use of a composition containing amino acids according to any one of claims 1-13 to reduce the epidermal aging process through modulation of the IL-6 expression and reduction of epidermal keratinization.

15. A cosmetic use of a composition containing amino acids according to any one of claims 1-13 to stimulate collagen and/or elastin production in the epidermis.

16. The composition according to any one of claims 1-13 for use in treating an inflammatory condition in a subject.

Europäisches
Patentamt

European
Patent Office

Office européen
des brevets

## EUROPEAN SEARCH REPORT

**Application Number**

EP 25 19 0633

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2014/209910 A1 (SAND BRUCE J [US]) 31 December 2014 (2014-12-31) * claims 1-32 * | 1-15 | INV. A61K8/44 A61Q19/08 A61K31/198 |
| X | WO 2019/195594 A1 (UNIV FLORIDA [US]) 10 October 2019 (2019-10-10) * claims 1-26 * * paragraphs [0142], [0144], [0151] * * figures 2A, 2B; example 2 * | 16 | A61P17/00 A61P29/00 |
| X | WO 2015/199753 A1 (SAND BRUCE J [US]) 30 December 2015 (2015-12-30) * claims 1-5 * * paragraphs [0138] - [0153] * | 1-15 | |
| X | HITOSHI MURAKAMI ET AL: "Importance of amino acid composition to improve skin collagen protein synthesis rates in UV-irradiated mice", AMINO ACIDS ; THE FORUM FOR AMINO ACID AND PROTEIN RESEARCH, SPRINGER-VERLAG, VI, vol. 42, no. 6, 23 August 2011 (2011-08-23), pages 2481-2489, XP035055882, ISSN: 1438-2199, DOI: 10.1007/S00726-011-1059-Z * abstract * * table 1 * | 1-15 | |

-----

-----

-----

-----

-/--

**TECHNICAL FIELDS SEARCHED (IPC)**

A61K
A61P
A61Q

The present search report has been drawn up for all claims

| Place of search | Date of completion of the search | Examiner |
|---|---|---|
| Munich | 28 November 2025 | Hörtner, Michael |

EPO FORM 1503 03.82 (P04C01)

page 1 of 2

Europäisches
Patentamt

European
Patent Office

Office européen
des brevets

## EUROPEAN SEARCH REPORT

Application Number

EP 25 19 0633

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | YAMANE TAKUMI ET AL: "Branched-chain amino acids regulate type I tropocollagen and type III tropocollagen syntheses via modulation of mTOR in the skin", BIOSCIENCE, BIOTECHNOLOGY, AND BIOCHEMISTRY , vol. 82, no. 4 3 April 2018 (2018-04-03), pages 611-615, XP093242959, JP ISSN: 0916-8451, DOI: 10.1080/09168451.2017.1386084 Retrieved from the Internet: URL:http://academic.oup.com/bbb/article-pdf/82/4/611/36844169/bbb0611.pdf * abstract * * figures 3, 4 * | 1-16 | |

-----

TECHNICAL FIELDS
SEARCHED      (IPC)

The present search report has been drawn up for all claims

| Place of search | Date of completion of the search | Examiner |
|---|---|---|
| Munich | 28 November 2025 | Hörtner, Michael |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

............................................................................................
& : member of the same patent family, corresponding document

EPO FORM 1503 03.82 (P04C01)

page 2 of 2

## ANNEX TO THE EUROPEAN SEARCH REPORT
## ON EUROPEAN PATENT APPLICATION NO.

EP 25 19 0633

This annex lists the patent family members relating to the patent documents cited in the above-mentioned European search report.
The members are as contained in the European Patent Office EDP file on
The European Patent Office is in no way liable for these particulars which are merely given for the purpose of information.

28-11-2025

| Patent document cited in search report | | Publication date | Patent family member(s) | | Publication date |
|---|---|---|---|---|---|
| WO 2014209910 | A1 | 31-12-2014 | NONE | | |
| WO 2019195594 | A1 | 10-10-2019 | AU | 2019247840 A1 | 22-10-2020 |
| | | | BR | 112020020318 A2 | 05-01-2021 |
| | | | CA | 3096039 A1 | 10-10-2019 |
| | | | CN | 112188891 A | 05-01-2021 |
| | | | EP | 3773539 A1 | 17-02-2021 |
| | | | JP | 2021520351 A | 19-08-2021 |
| | | | KR | 20200139776 A | 14-12-2020 |
| | | | US | 2021145797 A1 | 20-05-2021 |
| | | | WO | 2019195594 A1 | 10-10-2019 |
| WO 2015199753 | A1 | 30-12-2015 | NONE | | |

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5198465 A

**Non-patent literature cited in the description**

- **ZORINA, A.** ; **ZORIN, V.** ; **KUDLAY, D.** ; **KOPNIN, P.** Age-Related Changes in the Fibroblastic Differon of the Dermis: Role in Skin Aging. *Int. J. Mol. Sci.*, 2022, vol. 23, 6135, https://doi.org/10.3390/ijms23116135
- **KOVACS EJ**. Aging, traumatic injury, and estrogen treatment. *Exp Gerontol.*, July 2005, vol. 40 (7), 549-55
- **PARK CH** ; **LEE MJ** ; **AHN J** ; **KIM S** ; **KIM HH** ; **KIM KH** ; **EUN HC** ; **CHUNG JH**. Heat shock-induced matrix metalloproteinase (MMP)-1 and MMP-3 are mediated through ERK and JNK activation and via an autocrine interleukin-6 loop. *J Invest Dermatol.*, December 2004, vol. 123 (6), 1012-9
- **AGGARWAL BB** ; **TOTPAL K** ; **LAPUSHIN R** ; **CHATURVEDI MM** ; **PEREIRA-SMITH OM** ; **SMITH JR**. Diminished responsiveness of senescent normal human fibroblasts to TNF-dependent proliferation and interleukin production is not due to its effect on the receptors or on the activation of a nuclear factor NF-kappa B. *Exp Cell Res.*, May 1995, vol. 218 (1), 381-8
- **TANG A** ; **GILCHREST BA**. Regulation of keratinocyte growth factor gene expression in human skin fibroblasts. *J Dermatol Sci.*, January 1996, vol. 11 (1), 41-50
- **DOBSON GP** ; **BIROS E** ; **LETSON HL** ; **MORRIS JL**. Living in a Hostile World: Inflammation, New Drug Development, and Coronavirus. *Front Immunol.*, 22 January 2021, vol. 11, 610131
- **IL'YASOVA D** ; **COLBERT LH** ; **HARRIS TB** ; **NEWMAN AB** ; **BAUER DC** ; **SATTERFIELD S** ; **KRITCHEVSKY SB**. Circulating levels of inflammatory markers and cancer risk in the health aging and body composition cohort. *Cancer Epidemiol Biomarkers Prev.*, October 2005, vol. 14 (10), 2413-8
- **SU H** ; **LEI C-T** ; **ZHANG C**. Interleukin-6 Signaling Pathway and Its Role in Kidney Disease: An Update. *Front. Immunol.*, 2017, vol. 8, 405
- **ATAIE-KACHOIE P** ; **POURGHOLAMI MH** ; **RICHARDSON DR** ; **MORRIS DL**. Gene of the month: Interleukin 6 (IL-6). *J Clin Pathol.*, 16 July 2014, vol. 67 (11), 932-7

- **KISHIMOTO T**. Interleukin-6: discovery of a pleiotropic cytokine. *Arthritis Research & Therapy*, 2006, vol. 8 (2), S2
- **RANA H** ; **TRUONG NR** ; **JOHNSON B** ; **BAHARLOU H** ; **HERBERT JJ** ; **KANDASAMY S et al.** Herpes simplex virus spreads rapidly in human foreskin, partly driven by chemokine-induced redistribution of Nectin-1 on keratinocytes. *PLoS Pathog*, 2024, vol. 20 (6), e1012267, https://doi.org/10.1371/-journal. ppat.1012267
- **WILEY, C.D.** ; **VELARDE, M.C.** ; **LECOT, P.** ; **LIU, S.** ; **SARNOSKI, E.A.** ; **FREUND, A.** ; **SHIRAKAWA, K.** ; **LIM, H.W.** ; **DAVIS, S.S.** ; **RAMANATHAN, A.** *Cell Metabolism*, 09 February 2016, vol. 23, 303-314, http://dx.doi.org/10.1016/j.cmet.2015.11.011
- **R.M. SALAMA** ; **M.A. OMAR**. Anti-aging effect of nifuroxazide on skin changes of aged male rat models via modulating immunoreactivity of IL-6/NF-κB/Caspase-3. *Morphologie*, 2023, vol. 107 (359), ISSN 1286-0115, 100605, https://doi.org/10.1016/j.morpho.2023.06.001
- **GOSAIN, A.** ; **DIPIETRO, L.A.** Aging and wound healing. *World J. Surg.*, 2004, vol. 28, 321-326
- **FRANCESCHI, C.** ; **BONAFE, M.** ; **VALENSIN, S.** ; **OLIVIERI, F.** ; **DE LUCA, M.** ; **OTTAVIANI, E.** ; **DE BENEDICTIS, G.** Inflamm-aging. An evolutionary perspective on immunosenescence. *Ann. NY Acad. Sci.*, 2000, vol. 908, 244-254
- **KOVACS JE**. Aging, traumatic injury, and estrogen treatment. *Experimental Gerontology*, 2005, vol. 40, 549-555
- **GENDRISCH F** ; **ESSER PR** ; **SCHEMPP CM** ; **WÖLFLE U**. Luteolin as a modulator of skin aging and inflammation. *Biofactors*, 25 December 2020, vol. 47 (2), 170-180
- **BOISMAL F** ; **SERROR K** ; **DOBOS G** ; **ZUELGARAY E** ; **BENSUSSAN A** ; **MICHEL L**. Vieillissement cutané - Physiopathologie et therapies innovantes [Skin aging: Pathophysiology and innovative therapies. *Med Sci (Paris)*, 09 December 2020, vol. 36 (12), 1163-1172
- **JIN T** ; **LI L** ; **SIOW RC et al.** Collagen matrix stiffness influences fibroblast contraction force. *Biomed Phys Eng Express*, 2016, vol. 2, 047002

- **SRIRAM R** ; **GOPAL V**. Aging Skin and Natural Bioactives that Impede Cutaneous Aging: A Narrative Review. *Indian J Dermatol.*, July 2023, vol. 68 (4), 414-424

- **MOSMANN, T.** Rapid colourimetric assay for cellular growth and survival: Application to proliferation and cytotoxicity assays. *Journal of Immunological Methods*, 1983, vol. 65 (1-2), 55-63
- **VAN MEERLOO J** ; **KASPERS GJ** ; **CLOOS J.** Cell sensitivity assays: the MTT assay. *Methods Mol Biol.*, 2011, vol. 731, 237-45